# EUROPÄISCHE PATENTSCHRIFT

(11) **EP 4 122 503 B1**
(45) Veröffentlichungstag und Bekanntmachung des Hinweises auf die Patenterteilung: **22.04.2026**
(21) Anmeldenummer: 22155075.9
(22) Anmeldetag: 03.02.2022
(51) Int. Cl.: A61L 9/01

(54) **VORRICHTUNG ZUR ABGABE VON FLÜCHTIGEN SUBSTANZEN IN DIE UMGEBUNG**
DEVICE FOR DISPENSING VOLATILE SUBSTANCES INTO THE ENVIRONMENT
DISPOSITIF DE DISTRIBUTION DE SUBSTANCES VOLATILES DANS L'ENVIRONNEMENT

(30) Priorität: 03.02.2021 DE 202021100549 U
(43) Veröffentlichungstag der Anmeldung: 25.01.2023
(73) Patentinhaber: Mantz GmbH, 42651 Solingen (DE)
(72) Erfinder: SEEFELD, Jörg, 42651 Solingen (DE)
(74) Vertreter: Kudla, Christof

(56) Entgegenhaltungen:
- EP-A2- 0 381 529
- WO-A1-2011/123723
- WO-A1-2018/091686
- WO-A1-99/09120
- WO-A1-99/45971
- DE-A1- 102019 115 635
- US-A1- 2019 343 977

## Beschreibung

Die vorliegende Erfindung betrifft ein Kit zur Herstellung einer Vorrichtung zum Freisetzen eines Wirkstoffes in die Umgebung, umfassend oder bestehend aus a) eine Zusammensetzung bestehend aus oder umfassend a1) ein mit Wasser mischbares Lösungsmittel, a2) ein oder mehrere Wirkstoffe und b) mindestens ein Diffusionsstäbchen bestehend aus oder umfassend i) Fasern pflanzlicher Herkunft und/oder ii) Fasern tierischer Herkunft und/oder iii) einen Kunststoff. Die vorliegende Erfindung betrifft zudem eine Vorrichtung zum Freisetzen eines Wirkstoffes in die Umgebung bestehend aus oder umfassend a) eine flüssige Zusammensetzung bestehend aus oder umfassend a1) ein mit Wasser mischbares Lösungsmittel, a2) ein oder mehrere Wirkstoffe und a3) Wasser, b) mindestens ein Diffusionsstäbchen bestehend aus oder umfassend bestehend aus oder umfassend i) Fasern pflanzlicher Herkunft und/oder ii) Fasern tierischer Herkunft und/oder iii) einen Kunststoff und c) einen Behälter, der zumindest eine Behälteröffnung aufweist und die flüssige Zusammensetzung umfasst. Die vorliegende Erfindung betrifft zudem ein Verfahren zur Herstellung einer Vorrichtung zum Freisetzen eines Wirkstoffes wie die Verwendung von Diffusionsstäbchen bestehend aus oder umfassend i) Fasern pflanzlicher Herkunft und/oder ii) Fasern tierischer Herkunft in Verdampfersystemen. Die vorliegende Erfindung betrifft zudem eine Verwendung von Diffusionsstäbchen in Vorrichtungen (1) zum Freisetzen eines Wirkstoffes

Zur Beduftung und Erfrischung von Räumen werden neben Versprühsystemen häufig auch Verdunstungssysteme verwendet. Diese Verdunstungssysteme haben im Vergleich mit Versprühsystemen den Vorteil, dass sie einen über die gesamte Laufzeit annähernd konstanten Duft erzeugen, während bei Versprühsystemen lediglich ein intensiver Duft nach jeweils einem Sprühstoß erzeugt wird, der allmählich abnimmt.

Verdampfungssysteme bestehen in der Regel aus einem Behälter, der einen in einem organischen Lösungsmittel gelösten Duftstoff umfasst und ein in dem Behälter enthaltenden Diffusionsstäbchen. Die Diffusionsstäbchen sind dabei so ausgebildet, dass sie aufgrund von Kapillarkräften die organischen Lösungsmittel und den in dem Lösungsmittel gelösten Duftstoff aufnehmen und entlang des Diffusionsstäbchens aus dem Behälter befördern. An der Oberfläche der Diffusionsstäbchen verdampft das organische Lösungsmittel samt Duftstoff anschließend und der Duftstoff (nebst Lösungsmittel) wird in die Umgebung abgegeben.

Die im Stand der Technik beschriebenen Verdampfungssysteme weisen üblicherweise den Nachteil auf, dass die Zusammensetzung, die das organische Lösungsmittel und den in dem organischen Lösungsmittel gelösten Duftstoff enthält, immer als einsatzfertige Mischung verkauft werden. Es besteht nämlich kaum die Möglichkeit, dass der Verbraucher die Verdampfervorrichtung selbst finalisiert, indem er aus einem Konzentrat durch Verdünnen mit einem organischen Lösungsmittel die einsatzbereite Zusammensetzung erhält. Dem Verbraucher stehen nämlich nicht die benötigten organischen Lösungsmittel ohne Weiteres zur Verfügung, sodass es bisher üblich ist, einsatzfähige Mischungen anzubieten. Hierdurch weisen die Verdampfungssysteme ein hohes Gewicht (und Packmaß) auf und können sich nachteilig auf die Ökobilanz während der Herstellung, des Transportes und der Lagerung auswirken.

Zudem weisen die im Stand der Technik beschriebenen Verdampfungssysteme den Nachteil auf, dass sie einen hohen Anteil an flüchtigen organischen Verbindungen (englisch volatile organic compounds, kurz VOC) aufweisen, d.h. organische Stoffe, die bei Raumtemperatur oder höheren Temperaturen durch Verdampfen (umgangssprachlich "Verdunsten") in die Gasphase übergehen, also flüchtig sind. Während das Verdampfens der Aromastoffe bzw. anderen Wirkstoffe des Verdampfungssystems erwünscht ist, stellt das Verdampfen der eingesetzten Lösungsmittel lediglich ein bisher notwendiges Übel dar. Die meist geruchslosen oder kaum riechenden Lösungsmittel tragen zur Beduftung und Erfrischung von Räumen nicht bei, steigern allerding den Anteil der flüchtigen organischen Verbindungen in der Raumluft. Zudem sind die organischen Lösungsmittel meist brennbar und erhöhen die Brandgefahr.

WO 2018/091686 offenbart einen Lufterfrischer mit Docht aus Zellulose.

Aufgabe der vorliegenden Erfindung war es, eine Verdampfungssystem zur Verfügung zu stellen, bei dem
a) das Gewicht der Duftzusammensetzung reduziert werden kann, um die für die Herstellung, Lagerung und Transport benötigte Energie zu reduzieren und somit eine verbesserte Ökobilanz zu erreichen
   und/oder
b) den Anteil an flüchtigen organischen Verbindungen, die während der Verwendung der Verdampfervorrichtung freigesetzt werden zu reduzieren.

Diese Aufgabe wurde gelöst durch ein Kit zur Herstellung einer Vorrichtung (1) zum Freisetzen eines Wirkstoffes in die Umgebung, gemäß Anspruch 1.

Aus einem erfindungsgemäßen Kit kann eine Vorrichtung zum Freisetzen eines Wirkstoffes in die Umgebung (auch Verdampfungssysteme genannt) hergestellt werden, indem die Zusammensetzung des Kits in einen Behälter gegeben wird und zu dieser Zusammensetzung (5) eine definierte Menge Wasser hinzugegeben wird. Anschließend müssen die Diffusionsstäbchen (3, 3', 3") so in die resultierende, Wasser enthaltende Zusammensetzung gesteckt werden, dass ein Ende der Diffusionsstäbchen in die Zusammensetzung getaucht sind und sich die anderen Enden der Diffusionsstäbchen aus dem Behälter, der die Zusammensetzung enthält, erstrecken. In der resultierenden Vorrichtung zum Freisetzen eines Wirkstoffes besteht die Zusammensetzung (5) dann aus oder umfasst a1) ein mit Wasser mischbares Lösungsmittel, a2) ein oder mehrere Wirkstoffe und a3) Wasser. Hierdurch besteht die Möglichkeit, dass der Verbraucher durch Hinzufügen von Wasser aus der Zusammensetzung des erfindungsgemäßen Kits eine gebrauchsfertige Zusammensetzung erhält, die in einer Vorrichtung zum Freisetzen eines Wirkstoffs verwendet werden kann. Da die hergestellte Zusammensetzung üblicherweise bis zu 90 Gew.-% Wasser enthalten kann, ist somit eine Gewichts- und Volumeneinsparung von bis zu 90 % (teilweise sogar mehr) möglich. Herstellung einer Vorrichtung (1) zum Freisetzen eines Wirkstoffes aus einem erfindungsgemäßen Kit erfolgt somit durch das Zusetzen von Wasser, das dem Verbraucher immer zur Verfügung steht. Es ist nicht notwendig, dass der Verbraucher organische Lösungsmittel bevorratet und diese verwendet.

Während der Verwendung des resultierenden Verdampfungssystems wird zudem der Anteil an flüchtigen organischen Verbindungen signifikant reduziert, da statt eines organischen Lösungsmittels üblicherweise bis zu 90 Gew.-% Wasser eingesetzt werden.

Unter einem mit Wasser mischbaren Lösungsmittel wird im Rahmen der vorliegenden Erfindung ein Lösungsmittel verstanden, das bei einer Temperatur von 25 °C in jedem Verhältnis mit Wasser gemischt werden kann und hierbei vollständig eine homogene Phase ausbildet.

Ein erfindungsgemäßes Kit ist besonders bevorzugt, das zusätzlich c) einen Behälter (2) umfasst, der zumindest eine Behälteröffnung (4) aufweist. Hierbei ist es erfindungsgemäß bevorzugt, wenn die Zusammensetzung des Kits bereits in dem Behälter (2) enthalten ist. Der Verbraucher kann dann Wasser in den Behälter geben, um eine gebrauchsfertige Zusammensetzung zu erhalten, wobei vorzugsweise eine entsprechende Markierung in dem Behälter vorhanden ist, bis zur der das Wasser aufgefüllt werden muss. Alternativ besteht natürlich die Möglichkeit, dass dem Kit kein entsprechender Behälter zugegeben wird und der Verbraucher ein eigenes Behältnis verwendet oder einen in einem vorherigen Kit enthaltenen Behälter (wieder)verwendet. Sofern das Kit keinen entsprechenden Behälter umfasst, der eine Behälteröffnung (4) aufweist und ein Ausreichendes Innenvolumen aufweist, das die Zusammensetzung nebst Wasser aufnehmen kann, wird die Zusammensetzung vorzugsweis in einem kleineren Behälter, aus Keramik, Metall, Glas oder Kunststoffbehälter, oder einem Beutel abgefüllt.

Ein weiterer Aspekt der vorliegenden Erfindung betrifft eine Vorrichtung (1) zum Freisetzen eines Wirkstoffes in die Umgebung, vorzugsweise hergestellt aus einem erfindungsgemäßen Kit, bestehend aus oder umfassend
a) eine flüssige Zusammensetzung (5) bestehend aus oder umfassend
   a1) ein mit Wasser mischbares Lösungsmittel
   a2) ein oder mehrere Wirkstoffe
      und
   a3) Wasser
b) mindestens ein Diffusionsstäbchen (3, 3', 3") bestehend aus oder umfassend
   i) Fasern pflanzlicher Herkunft
      und/oder
   ii) Fasern tierischer Herkunft
      und/oder
   iii) einen Kunststoff.
      und
c) einen Behälter (2), der zumindest eine Behälteröffnung (4) aufweist und die flüssige Zusammensetzung (5) umfasst.

Es ist erfindungsgemäß bevorzugt, wenn der Behälter (2) ein Glasbehälter, Keramikbehälter oder Metallbehälter ist. Diese Behälter haben den Vorteil, dass sie ausgewaschen und wiederverwendet werden können und dabei keine Gerüche aufnehmen. Zudem besteht bei diesen Materialine nicht die Gefahr, dass es zu einer Verfärbung der Flasche kommt. Alternativ zu Glas besteht auch die - weniger bevorzugte - Möglichkeit, dass ein Behälter aus einem Kunststoff, vorzugsweise einem transparenten Kunststoff verwendet wird. Erfindungsgemäß können somit Behälter aus Glas, Keramik, Metall oder Kunststoff verwendet werden. Es ist erfindungsgemäß bevorzugt, wenn der Behälter ein Füllvolumen (Innenvolumen) im Bereich von 25 bis 250 mL aufweist, vorzugsweise im Bereich von 40 bis 220 mL. Üblicherweise werden Behälter mit einem Füllvolumen von 50 mL ± 15 mL, 90 mL ± 15 mL, 100 mL ± 15 mL, 120 mL ± 15 mL, 200 mL ± 15 mL eingesetzt oder 250 mL ± 15 mL eingesetzt.

Ein weiterer Aspekt der vorliegenden Erfindung betrifft eine Zusammensetzung bestehend aus oder umfassend
a1) ein mit Wasser mischbares Lösungsmittel
a2) ein oder mehrere Wirkstoffe
   und
a3) optional Wasser.

Sofern nachfolgend lediglich eine Zusammensetzung erwähnt wird, kann es sich sowohl um eine Zusammensetzung des erfindungsgemäßen Kits, eine flüssige Zusammensetzung der erfindungsgemäßen Vorrichtung (1) als auch eine erfindungsgemäße Zusammensetzung handeln. Ansonsten wird zwischen einer Zusammensetzung des erfindungsgemäßen Kits und einer Zusammensetzung der erfindungsgemäßen Vorrichtung (1) unterschieden.

Es ist erfindungsgemäß bevorzugt, wenn die Zusammensetzung zusätzlich a4) ein Tensid enthält. Insbesondere bei einem hohen Wasseranteil kann es notwendig werden, dass ein Tensid zugesetzt wird, um eine Emulsion aus Wirkstoff und Lösungsmittel (a1) das mit Wasser mischbares Lösungsmittel und a3) Wasser) herzustellen. Ob ein Tensid verwendet werden muss, hängt von der Löslichkeit des Wirkstoffes in Wasser ab.

Eigenen Untersuchungen haben ergeben, dass es erfindungsgemäß bevorzugt ist, wenn das Tensid einen nach Griffin berechneten HLB-Wert im Bereich von 3 bis 20 aufweist, vorzugsweise im Bereich von 6 bis 20, besonders bevorzugt im Bereich von 9 bis 20.

Erfindungsgemäß bevorzugt handelt es sich bei dem Tensid um Tenside ausgewählt aus der Gruppe bestehend aus nichtionischen Tensiden, anionischen Tensiden, kationischen Tensiden und amphotere Tenside.

Erfindungsgemäß besonders bevorzugt handelt es sich bei dem nichtionischen Tensid um eine Verbindung, die mehrere Alkohol-Gruppen aufweist, um eine Verbindung, die eine oder mehrere Ether-Gruppen aufweist, oder um eine Verbindung, die sowohl Alkohol-Gruppen als auch Ether-Gruppen aufweisen (z. B. Ethoxylate).

Erfindungsgemäß besonders bevorzugt handelt es sich bei dem anionischen Tensid um eine Verbindung, die eine oder mehrere Carboxylat-Gruppen aufweist, um eine Verbindung, die eine oder mehrere Sulfonat-Gruppen aufweist, oder um eine Verbindung, die eine oder mehrere Sulfat-Gruppen aufweist.

Erfindungsgemäß besonders bevorzugt handelt es sich bei dem kationischen Tensid um eine Verbindung, die eine oder mehrere quartäre Ammonium-Gruppen aufweist.

Erfindungsgemäß besonders bevorzugt handelt es sich bei dem amphoteren Tensid um eine Verbindung, die sowohl Carboxylat-Gruppen als auch quartäre Ammonium-Gruppen aufweisen.

Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen.

Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Alkylethersulfate, Glyce- rinethersulfate, Fettsäureethersulfate, Hydroxymischethersulfate, Monoglycerid- (ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N- Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate, Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisothionate, Alkylphosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid- oder Propylenoxideinheiten, bevorzugt 1 bis 3 Ethylenoxideinheiten, im Molekül aufweisen. Dazu zählen z. B. Natriumlaurylsulfat, Ammoniumtaurytsulfat, Natriumlaurylethersulfat, Ammoniumlaurylethersulfat, Natriumlaurylsarkosinat, Natriumoleylsuccinat, Ammoniumlaurylsulfosuccinat, Natriumdodecylbenzolsulfonat, Triethanolamindodecylbenzolsulfonat.

Sofern die anionischen Tenside Polyglycoletherketten enthalten, können diese eine konventionelle, vorzugsweise jedoch eine eingeengte Homologenverteilung aufweisen.

Typische Beispiele für kationische Tenside sind quartäre Ammoniumverbindungen, wie beispielsweise das Cetyltrimethylammoniumchlorid, Dimethyldistearylammoniumchlorid, und Esterquats, insbesondere quaternierte Fettsäuretrialkanolaminestersalze.

Geeignete amphotere Tenside sind z. B. Aminopropionate, Aminoglycinate, Imidazoliniumbetaine und Sulfobetaine, Alkylbetaine, Alkylamidopropylbetaine, Alkylsulfobetaine, Alkylglycinate, Alkylcarboxyglycinate, Alkylamphoacetate oder -Propionate, Alkylamphodiacetate oder -dipropionate.

Es ist erfindungsgemäß bevorzugt, wenn der Wirkstoff bzw. die Wirkstoffe Duftstoffe, antimikrobielle Wirkstoffe und/oder Insektenrepellentien sind. Hierbei ist es allerdings nicht ausgeschlossen, dass beispielsweise einige Duftstoffe auch gleichzeitig eine Wirkung gegen Insekten aufweisen, wie dies beispielsweise bei Citronellol und Geraniol der Fall ist. Erfindungsgemäß besonders bevorzugt handelt es sich bei dem Wirkstoff um einen Duftstoff.

Im Rahmen dieses Textes wird unter Duftstoffen eine Substanz oder ein Substanzgemisch verstanden, das olfaktorisch wahrgenommen wird.

Die Auswahl der Duftstoffe ist dabei sehr umfassend; entsprechende Duftstoffe, die erfindungsgemäß eingesetzt werden können finden sich z. B. in "S. Arctander, Perfume and Flavor Chemicals, Vol. I und II, Montclair, N. J., 1969, Selbstverlag" oder "H. Surburg und J. Panten, Common Fragrance and Flavor Materials, 6th ed., Wiley-VCH, Weinheim, 2016". Im Einzelnen seien im Folgenden Duftstoffe genannt, ausgewählt aus der Gruppe
der Kohlenwasserstoffe, dabei bevorzugt 3-Caren; α-Pinen; β-Pinen; α,-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;
der aliphatischen Alkohole, dabei bevorzugt Hexanol; Octanol; 3-Octanol; 2,6-Dimethylheptanol; 2-Methyl-2-heptanol; 2-Methyl-2-octanol; (E)-2-Hexenol; (E)- und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol; 3,7-DIMETHYL-6-OCTEN-1-OL;
der aliphatischen Aldehyde und deren Acetale, dabei bevorzugt Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal;2,6,10-Trimethyl-9-undecenal; 2,6,10-Trimethyl-5,9-undecadienal; Heptanaldiethylacetal; 1,1-Dimethoxy-2,2,5-trimethyl-4-hexen; Citronellyloxyacetaldehyd; 1-(1-Methoxy-propoxy)-(E/Z)-3-hexen;
der aliphatischen Ketone und deren Oxime, dabei bevorzugt 2-Heptanon; 2-Octanon; 3-Octanon; 2-Nonanon; 5-Methyl-3-heptanon; 5-Methyl-3-heptanonoxim; 2,4,4,7-Tetramethyl-6-octen-3-on; 6-Methyl-5-hepten-2-on; 1-(1,2,3,4,5,6,7,8-OCTAHYDRO-2,3,8,8-TETRAMETHYL-2-NAPHTHYL)ETHAN-1-ONE; 3-METHYL-4-(2,6,6-TRIMETHYL-2-CYCLOHEXEN-1-YL)-3-BUTEN-2-ONE
der aliphatischen schwefelhaltigen Verbindungen, dabei bevorzugt 3-Methylthio-hexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
der aliphatischen Nitrile, dabei bevorzugt 2-Nonensäurenitril; 2-Undecensäurenitril; 2-Tridecensäurenitril; 3,12-Tridecadiensäurenitril; 3,7-Dimethyl-2,6-octadien-säurenitril; 3,7-Dimethyl-6-octensäurenitril;
der Ester von aliphatischen Carbonsäuren, dabei bevorzugt (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat; Isoamylbutyrat; Hexylbutyrat; (E)- und (Z)-3-Hexenyl-isobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat;4-Methyl-2-pentyl-crotonat;
der acyclischen Terpenalkohole, dabei bevorzugt Citronellol; Geraniol; Nerol; Linalool; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate und 3-Methyl-2-butenoate;
der acyclischen Terpenaldehyde und -ketone, dabei bevorzugt Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;
der cyclischen Terpenalkohole, dabei bevorzugt Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate und 3-Methyl-2-butenoate; Menthylformiat; Menthylpropionat; Menthylbutyrat; Menthylisobutyrat; Menthylisovalerianat; Menthylhexanoat; Menthylcrotonat; Menthyltiglinat;
der cyclischen Terpenaldehyde und -ketone, dabei bevorzugt Menthon; Isomenthon; 8-Mercaptomenthan-3-on; Carvon; Campher; Fenchon; alpha-Ionon; beta-Ionon; beta-n-Methylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on;2-Methyl-4-(2,6,6-trimethyl-1-cyclohexen-1-yl)-2-butenal; Nootkaton; Dihydro-nootkaton; 4,6,8-Megastigmatrien-3-on; alpha-Sinensal; beta-Sinensal; acetyliertes Ce-dernholzöl (Methylcedrylketon);
der cyclischen Alkohole, dabei bevorzugt 4-tert.-Butylcyclohexanol; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
der cycloaliphatischen Alkohole, dabei bevorzugt alpha,3,3-Trimethylcyclohexylmethanol; 1-(4-Isopropylcyclohexyl)ethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
der cyclischen und cycloaliphatischen Ether, dabei bevorzugt Cineol; Cedrylmethylether; Cyclododecylmethylether; 1,1-Dimethoxycyclododecan; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9- Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
der cyclischen und makrocyclischen Ketone, dabei bevorzugt 4-tert.-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 8-Cyclohexadecen-1-on; 9-Cycloheptadecen-1-on; Cyclopentadecanon; Cyclohexadecanon;
der cycloaliphatischen Aldehyde, dabei bevorzugt 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
der cycloaliphatischen Ketone, dabei bevorzugt 1-(3,3-Dimethylcyclohexyl)-4-penten-1-on; 2,2-Dimethyl-1-(2,4-dimethyl-3-cyclohexen-1-yl)-1-propanon; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
der Ester cyclischer Alkohole, dabei bevorzugt 2-tert-Butylcyclohexylacetat; 4-tert-Butylcyclohexylacetat; 2-tert-Pentylcyclohexylacetat; 4-tert-Pentylcyclohexylacetat; 3,3,5-Trimethylcyclohexylacetat; Decahydro-2-naphthylacetat; 2-Cyclopentylcyclopentylcrotonat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat;
der Ester cycloaliphatischer Alkohole, vorzugsweise 1-Cyclohexylethylcrotonat;
der Ester cycloaliphatischer Carbonsäuren, dabei bevorzugt Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
der araliphatischen Alkohole, dabei bevorzugt Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentan-1-ol; 3-Methyl-5-phenylpentanol; 3-Phenyl-2-propen-1-ol; 4-Methoxybenzylalkohol; 1-(4-Isopropylphenyl)ethanol;
der Ester von araliphatischen Alkoholen und aliphatischen Carbonsäuren, dabei bevorzugt Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat;
der araliphatischen Ether, dabei bevorzugt 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxan; 4,4a,5,9b-Tetrahydroindeno[1,2-d]-m-dioxin; 4,4a,5,9b-Tetrahydro-2,4-dimethylindeno[1,2-d]-m-dioxin;
der aromatischen und araliphatischen Aldehyde, dabei bevorzugt Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 2-Methyl-3-(4-isobutylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatischen Ketone, dabei bevorzugt Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon;2-Benzofuranylethanon;(3-Methyl-2-benzofuranyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1,1,2,6-tetramethyl-3-(1-methylethyl)-1H-5-indenyl]ethanon; 5',6',7',8'-Tetrahydro-3',5',5',6',8',8'-hexamethyl-2-acetonaphthon;
der aromatischen und araliphatischen Carbonsäuren und deren Ester, dabei bevorzugt Benzoesäure; Phenylessigsäure; Methylbenzoat; Ethylbenzoat; Hexylbenzoat; Methylphenylacetat; Ethylphenylacetat; Geranylphenylacetat; Phenylethyl-phenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen, dabei bevorzugt 2,4,6-Trinitro-1,3-dimethyl-5-tert.-butylbenzol; 3,5-Dinitro-2,6-dimethyl-4-tert.-butylacetophenon; Zimtsäurenitril; 3-Methyl-5-phenyl-2-pentensäurenitril; 3-Methyl-5-phenylpentansäurenitril; Methylanthranilat; Methy-N-methylanthranilat; Schiff'sche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropylchinolin; 6-Isobutylchinolin; 6-sec.-Butylchinolin;2-(3-Phenylpropyl)pyridin; Indol; Skatol; 2-Methoxy-3-isopropylpyrazin; 2-Isobutyl-3-methoxypyrazin;
der Phenole, Phenylether und Phenylester, dabei bevorzugt Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen, dabei bevorzugt 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone, dabei bevorzugt 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid;4-Methyl-1,4-decanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,13-tridecandioat; Cumarin; 2,3-Dihydrocumarin; Octahydrocumarin.

Bei den erfindungsgemäß verwendeten Duftstoffen kann es sich ebenfalls um ätherische Öle, Concretes, Absolues, Resine, Resinoide, Balsame und/oder Tinkturen handeln. Bevorzugte Etherische Öle, Concretes, Absolues, Resine, Resinoide, Balsame und/oder Tinkturen sind bevorzugt auszuwählen aus der Gruppe bestehend aus:
Ambratinktur; Amyrisöl; Angelicasamenöl; Angelicawurzelöl; Anisöl; Baldrianöl; Basilikumöl; Baummoos-Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cade-öl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-Öl; Eucalyptusöl; Fenchelöl; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl; Lavendel-Absolue; Lavendelöl; Lemongrasöl; Liebstocköl; Limetteöl destilliert; Limetteöl gepreßt; Linaloeöl; Litseacubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamen-öl; Petitgrainöl; Pfefferminzöl; Pfefferöl; Pimentöl; Pineöl (engl.: pine oil); Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetes-öl; Tannennadelöl; Teebaum-Öl; Terpentinöl (engl.: turpentine oil); Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl.

Ein erfindungsgemäßes Kit ist besonders bevorzugt, dass zusätzlich d) eine Verpackung umfasst, die die Komponenten a, b und ggf. c umfasst bzw. aufnimmt. Insbesondere im Verkauf ist es vorteilhaft, wenn die einzelnen Komponenten entsprechend verpackt sind. Vorzugsweise besteht die Verpackung aus Papier, Karton oder Pappe oder umfasst diese Stoffe.

Es ist erfindungsgemäß bevorzugt, wenn das mit Wasser mischbare Lösungsmittel ein Alkohol oder ein Ether ist, vorzugsweise Ethanol oder Dipropylenglycolmethylether ist. Eigene Untersuchungen haben gezeigt, dass die Verwendung von Alkoholen oder Ethern, insbesondere Ethanol oder Dipropylenglycolmethylether besonders gute Eigenschaften in erfindungsgemäßen Vorrichtungen zeigen. Sowohl Ethanol als auch Dipropylenglycolmethylether sind mit Wasser mischbar und weisen einen ausreichenden Dampfdruck bei Raumtemperatur auf.

In einem erfindungsgemäßen Kit ist es bevorzugt, wenn die Zusammensetzung des erfindungsgemäßen Kits flüssig ist und sämtliche Komponenten der Zusammensetzung gelöst sind, d.h. das eine homogene Phase ausgebildet ist. Es ist auch möglich - wenngleich weniger bevorzugt - wenn einzelne Komponenten der Zusammensetzung des erfindungsgemäßen Kits nicht oder nur teilweise gelöst sind und (teilweise) als Feststoff vorliegen. In diesem Fall ist es allerdings bevorzugt, wenn nach der Zugabe von Wasser zu der Zusammensetzung des erfindungsgemäßen Kits währen der Herstellung der Zusammensetzung der erfindungsgemäßen Vorrichtung alle Feststoffe gelöst werden und eine homogene Phase ausgebildet wird. In einer erfindungsgemäßen Vorrichtung ist es bevorzugt, wenn die Zusammensetzung der erfindungsgemäßen Vorrichtung flüssig ist und sämtliche Komponenten der Zusammensetzung gelöst sind, d.h. dass eine homogene Phase ausgebildet ist. Abhängig von den eingesetzten Duftstoffen besteht auch die Möglichkeit, dass die Duft- bzw. Wirkstoffe in dem Gemisch aus Wasser und dem mit Wasser mischbaren Lösungsmittel emulgiert vorliegen oder ein Teil der Duft- bzw. Wirkstoffe emulgiert und ein Teil gelöst vorliegen. In diesem Fall ist es erfindungsgemäß bevorzugt, wenn ein heterogenes Gemisch vorliegt.

Erfindungsgemäß sind Diffusionsstäbchen bevorzugt, bei denen die Fasern pflanzlicher Herkunft ausgewählt sind aus der Gruppe bestehend aus Zellstofffasern, Samenfasern, Bastfasern und Hartfasern. Besonders bevorzugt ist es hierbei, wenn es sich bei den Fasern pflanzlicher Herkunft um Zellstofffasern handelt.

Die Verwendung Diffusionsstäbchen, die Fasern pflanzlicher Herkunft umfassen oder aus Fasern pflanzlicher Herkunft bestehen, hat den Vorteil, dass die Diffusionsstäbchen nach der Verwendung dem Papierrecycling zugesetzt werden können und vollständig biologisch abgebaut werden können. So besteht sogar die Möglichkeit, dass entsprechende Diffusionsstäbchen dem Kompost zugeführt werden können. Anders als bei der Verwendung von Diffusionsstäbchen, die Kunststoffe umfassen, lösen sich die Diffusionsstäbchen, die pflanzliche Fasern umfassen, in ihre Bestandteile auf, sind damit biologisch abbaubar und belasten die Umwelt nicht.

Erfindungsgemäß sind Diffusionsstäbchen bevorzugt, die Fasern pflanzlicher Herkunft und keine Kunststoffe umfassen.

Im Rahmen der vorliegenden Erfindung wird unter Holz oder anderen Pflanzenteilen kein Material verstanden, das pflanzliche Faser oder Zellstofffasern umfasst oder daraus besteht. Unter pflanzlichen Fasern bzw. Zellstofffasern wird im Rahmen der vorliegenden Erfindung eine Faser verstanden, die nach einer mechanischen und/oder chemischen Aufarbeitung von Holz oder anderen Pflanzenteilen resultiert, wie sie beispielsweise in der Papierherstellung üblich ist.

Hierbei ist es erfindungsgemäß bevorzugt, wenn der Massenanteil an Zellstofffasern größer gleich 60,0 % beträgt, vorzugsweise größer gleich 70,0 % beträgt, besonders bevorzugt größer 75,0 % beträgt, bezogen auf die Gesamtmasse des Diffusionsstäbchen und bestimmt als lufttrockener Massenanteil (Lutro).

Ebenfalls ist es erfindungsgemäß bevorzugt, wenn der Massenanteil an Zellstofffasern kleiner gleich 90,0 % beträgt, vorzugsweise kleiner gleich 85 % beträgt, besonders bevorzugt kleiner gleich 80,0 % beträgt, bezogen auf die Gesamtmasse des Diffusionsstäbchen und bestimmt als lufttrockener Massenanteil (Lutro).

Es ist erfindungsgemäß bevorzugt, wenn der Massenanteil an Zellstofffasern im Bereich von 60,0 % bis 90,0 % liegt, vorzugsweise im Bereich von 70,0 % bis 85,0 % liegt, besonders bevorzugt im Bereich von 75,0 % bis 80,0 % liegt, bezogen auf die Gesamtmasse des Diffusionsstäbchen und bestimmt als lufttrockener Massenanteil (Lutro).

Der Wassergehalt bei lufttrockenen Diffusionsstäbchen liegt üblicherweise im Bereich von 1 bis 7 Gew.-%.

Es ist erfindungsgemäß bevorzugt, wen das mindestens ein Diffusionsstäbchen (3, 3', 3") oder sämtliche Diffusionsstäbchen ein Bindemittel umfasst, wobei es sich bei dem Bindemittel vorzugsweise um Stärke handelt. Bei der Stärke kann es sich um Stärke handeln ausgewählt aus der Gruppe bestehend aus natürlicher Stärke, anionischer Stärke, kationischer Stärke, peroxidierter Stärke, veretherter Stärke, veresterter Stärke, oxidierter Stärke, hydrolysierter Stärke, dextrinierter Stärke, hydroxyethylierter Stärke und acetylierter Stärke. Die Verwendung von natürlicher Stärke ist erfindungsgemäß besonders bevorzugt.

Hierbei ist es erfindungsgemäß bevorzugt, wenn der Massenanteil an Bindemittel im Bereich von 1,0 % bis 10,0 % liegt, vorzugsweise im Bereich von 2,0 % bis 8,0 % liegt, besonders bevorzugt im Bereich von 3,0 % bis 5,0 % liegt, bezogen auf die Gesamtmasse des Diffusionsstäbchen und bestimmt als lufttrockenen Massenanteil (Lutro).

Eigene Untersuchungen haben überraschenderweise gezeigt, dass Diffusionsstäbchen, die Zellstofffasern und Stärke als Bindemittel umfassen, besonders gute Transporteigenschaften für die Zusammensetzungen der Vorrichtung aufweisen. Üblicherweise wäre der Fachmann nämlich davon ausgegangen, dass Diffusionsstäbchen auf Basis von Zellstofffasern beim Kontakt Zusammensetzungen mit einem hohen Wasseranteil aufquellen und innerhalb von kurzer Zeit nicht mehr funktionstüchtig sind. Eigene Untersuchungen haben gezeigt, dass Diffusionsstäbchen, die aus Zellstofffasern und Stärke bestehen sehr gut mit wässrigen Zusammensetzungen verträglich sind.

Es ist erfindungsgemäß bevorzugt, dass das mindestens ein Diffusionsstäbchen (3, 3', 3") oder sämtliche Diffusionsstäbchen ein oder mehrere Füllmittel umfasst, wobei es sich bei dem Füllmittel vorzugsweise um Carbonate handelt.

Hierbei ist er erfindungsgemäß bevorzugt, wenn das oder die Carbonate ausgewählt sind aus der Gruppe bestehend aus Magnesiumcarbonat, Calciumcarbonat, Berylliumcarbonat, Lithiumcarbonat, Natriumcarbonat, Kaliumcarbonat und Calcium-Magnesium-Carbonat, vorzugsweise ausgewählt sind aus der Gruppe bestehend aus Magnesiumcarbonat, Calciumcarbonat und Calcium-Magnesium-Carbonat.

Hierbei ist es erfindungsgemäß bevorzugt, wenn der Massenanteil an Füllmittel im Bereich von 3,0 % bis 25,0 % liegt, vorzugsweise im Bereich von 5,0 % bis 20,0 % liegt, besonders bevorzugt im Bereich von 10,0 % bis 15,0 % liegt, bezogen auf die Gesamtmasse des Diffusionsstäbchen und bestimmt als lufttrockener Massenanteil (Lutro).

Eigene Untersuchungen haben gezeigt, dass bei der Verwendung von Füllstoffen und insbesondere bei der Verwendung von Carbonaten die physikalischen Eigenschaften der Diffusionsstäbchen noch weiter verbessert werden können. Hierdurch ist es möglich, dass die Diffusionsstäbchen über einen langen Zeitraum verwendet werden könne, selbst wenn die Hauptsächlich aus Zellstofffasern bestehen.

Es ist erfindungsgemäß bevorzugt, wenn es sich bei den Zellstofffasern um Nadelholz-, Laubholz-, Strohzellstofffasern oder Mischungen daraus handelt. Besonders bevorzugt ist es hierbei, wenn es sich bei den Zellstofffasern um Fasern aus Kiefern-, Birken, Pappeln, Fichten-, Buchen- oder Eukalyptusholz handelt. Eigene Untersuchungen haben gezeigt, dass diese Zellstofffasern besonders gute Eigenschaften beim Transport der Zusammensetzung der erfindungsgemäßen Vorrichtung aufweisen.

Es ist erfindungsgemäß bevorzugt, wenn das Diffusionsstäbchen als Zellstofffasern Frischfasern umfasst und der Massenanteil an Frischfasern größer gleich 80 % beträgt, bevorzugt größer gleich 90 %, besonders bevorzugt größer gleich 95 %, bezogen auf die Gesamtmasse der Zellstofffasern und bestimmt als lufttrockener Massenanteil (Lutro).

Es ist erfindungsgemäß bevorzugt, wenn sämtliche Zellstoffasern im Papiersubstrat Frischfasern sind, d.h. wobei der Massenanteil an Frischfasern 100 % beträgt, bezogen auf die Gesamtmasse der Zellstofffasern und bestimmt als lufttrockener Massenanteil (Lutro).

Es ist erfindungsgemäß bevorzugt, wenn das Diffusionsstäbchen als Zellstofffasern Recycling-Fasern umfasst und der Massenanteil an Recycling-Faser größer gleich 40 % beträgt, bevorzugt größer gleich 50 %, besonders bevorzugt größer gleich 60 %, bezogen auf die Gesamtmasse der Zellstofffasern und bestimmt als lufttrockener Massenanteil (Lutro).

Es ist erfindungsgemäß bevorzugt, wenn die Diffusionsstäbchen zusätzlich Hilfsstoffe umfasst ausgewählt aus der Gruppe anorganische Füllstoffe, Bindemittel (vorzugsweise Stärke), Alaun und/oder Leim.

In einer alternativen Ausgestaltung sind Diffusionsstäbchen bevorzugt, bei denen die Fasern tierischer Herkunft ausgewählt sind aus der Gruppe bestehend aus Wolle, feinen Tierhaaren, groben Tierhaaren und Seiden.

In einer alternativen Ausgestaltung sind Diffusionsstäbchen bevorzugt, bei denen die Fasern tierischer Herkunft ausgewählt sind aus der Gruppe bestehend aus Wolle, feinen Tierhaaren, groben Tierhaaren und Seiden.

In einer alternativen Ausgestaltung sind Diffusionsstäbchen bevorzugt, bei denen mindestens ein Diffusionsstäbchen (3, 3', 3") aus Kunststoff besteht oder Kunststoff umfasst, wobei es sich bei dem Polymer vorzugsweise um einen Polyester handelt, besonders bevorzugt, wobei es sich bei dem Polyester um Polyethylenterephthalat handelt.

Im Rahmen der vorliegenden Erfindung wird unter einem Polymer bzw. Kunststoff ein chemischer Stoff verstanden, der aus Makromolekülen besteht. Erfindungsgemäß besonders bevorzugt ist es, wenn es sich bei dem Polymer um ein synthetisches Polymer handelt, d.h. um ein Makromolekül, dass industriell oder im Labormaßstab hergestellt wurde. Erfindungsgemäß bevorzugt handelt es sich bei dem Polymer nicht um Cellulose oder Lignin.

Ebenfalls erfindungsgemäß bevorzugt handelt es sich bei dem Diffusionsstäbchen nicht um ein Diffusionsstäbchen, das Rattan, Bambus, Balsaholz, Peddingrohr oder ein anderes Holz umfasst oder daraus besteht.

Erfindungsgemäß bevorzugt ist es, wenn das mindestens eine Diffusionsstäbchen (3, 3', 3") aus Kunststoff besteht oder Kunststoff umfasst und ein Faser-Diffusionsstäbchen ist. Im Rahmen der vorliegenden Erfindung wird unter einem Faser-Diffusionsstäbchen ein Diffusionsstäbchen verstanden, dass aus einzelnen Fasern gebildet wird, die parallel zur Länge des Diffusionsstäbchens verlaufen. Zur Herstellung der Faser-Diffusionsstäbchen können die einzelnen Fasern beispielsweise miteinander verklebt oder thermisch und/oder durch Einwirkung von Druck miteinander verbunden, sodass sie ein festes Faser-Diffusionsstäbchen ausbilden. Durch die einzelnen Fasern bilden sich innerhalb des Diffusionsstäbchens Kapillarräume aus, die dazu geeignet sind Flüssigkeiten aufzunehmen und durch die wirkenden Kapillarkräfte zu fördern.

Es ist erfindungsgemäß bevorzugt, wenn das mindestens eine Diffusionsstäbchen (3, 3', 3") aus Kunststoff besteht oder Kunststoff umfasst und ein Faser-Diffusionsstäbchen ist und wobei das mindestens eine Diffusionsstäbchen ein Polyethylenterephthalat umfasst oder daraus besteht.

Ebenfalls erfindungsgemäß bevorzugt handelt es sich bei dem Diffusionsstäbchen nicht um ein Diffusionsstäbchen, das Glas oder Glasfasern (Glasfaser-Diffusionsstäbchen) umfasst oder daraus besteht.

Die Diffusionsstäbchen können mit einem Farbstoff eingefärbt sein. Hierbei ist darauf zu achten, dass der Farbstoff in dem Material des Diffusionsstäbchens, gebunden ist und ein Ausbluten des Farbstoffes in die flüssige Zusammensetzung nicht erfolgt. Entsprechende Farbstoffe sind dem Fachmann bekannt. Um die Optik der erfindungsgemäßen Vorrichtung zu verbessern, besteht die Möglichkeit unterschiedlich eingefärbte Diffusionsstäbchen zu verwenden. Im Rahmen der vorliegenden Erfindung werden unterschiedlich eingefärbte oder ausgestaltete Diffusionsstäbchen als zu einer einzigen Sorte angehörend verstanden, sofern das Grund-Material aus denen die Diffusionsstäbchen gebildet ist, identisch ist. So sind beispielsweise Diffusionsstäbchen aus Polyethylenterephthalat, die lediglich unterschiedlich eingefärbt sind oder andere übliche Polymeradditive enthält, allerdings ansonsten identisch ausgebildet sind, als eine einzige Sorte an Diffusionsstäbchen anzusehen.

Erfindungsgemäß bevorzugt sind die Diffusionsstäbchen zylindrisch ausgebildet. Allerdings besteht auch die Möglichkeit, dass andere Formen gebildet werden, so beispielsweise Quader. Die Stäbchen können auch andere Querschnitte, wie beispielsweise Herzen, Sterne, Schneeflocken, aufweisen.

Es ist erfindungsgemäß bevorzugt, wenn die Diffusionsstäbchen einen Durchmesser im Bereich von 2 bis 10 mm aufweisen, vorzugsweise im Bereich von 3 bis 7 mm aufweisen, besonders bevorzugt im Bereich von 3 bis 5 mm aufweisen. Üblicherweise werden Diffusionsstäbchen mit einem Durchmesser von 4 mm ± 0,5 mm eingesetzt.

Es ist erfindungsgemäß bevorzugt, wenn die Diffusionsstäbchen eine Länge im Bereich von 50 bis 240 mm aufweisen, vorzugsweise im Bereich von 80 bis 220 mm aufweisen, besonders bevorzugt im Bereich von 90 bis 200 mm aufweisen. Üblicherweise werden Diffusionsstäbchen mit einer Länge von 170 mm ± 20 mm, 200 mm ± 20 mm oder 240 mm± 20 mm eingesetzt.

Über die Länge, den Durchmesser und die Anzahl der Diffusionsstäbchen kann die Diffusions- und Verdunstungsrate der Zusammensetzung gesteuert werden. Üblicherweise werden drei, vier, fünf, sechs, sieben, acht oder neun Diffusionsstäbchen eingesetzt und sind in einem erfindungsgemäßen Kit vorhanden. Zudem wird die Anzahl, die Länge und der Durchmesser auch an die Menge bzw. Behältergröße angepasst.

Es ist erfindungsgemäß wenn die Diffusionsstäbchen durch Aufrollen von Papier hergestellt wurden.

Hierbei ist erfindungsgemäß bevorzugt, wenn die Diffusionsstäbchen eine hohle Seele entlang ihrer Achse aufweisen. In dieser Ausgestaltung handelt es sich bei dem Diffusionsstäbchen um eine Hohlzylinder mit einem (Außen)Durchmesser, einem Durchmesser der Seele (Innendurchmesser) und einer Wanddicke.

Überraschenderweise hat es sich gezeigt, dass Diffusionsstäbchen, die eine Seele entlang ihrer Achse aufweisen eine höhere Verdunstungsrate aufweisen. Ohne sich dabei auf eine Theorie festlegen zu wollen, wird davon ausgegangen, dass die Seele als Kapillare wirkt und den Transport der Zusammensetzung weiter verbessert. Wie allgemein bekannt ist die Kapillarwirkung einer Röhre vom Durchmesser der Kapillare abhängig. Eigene Untersuchungen haben dabei gezeigt, dass bei einem Durchmesser von weniger als 0,60 mm die Kapillarität so hoch ist, dass die gesamte Seele mit der Zusammensetzung gefüllt ist. Bei einem Durchmesser der Seele von mehr als 4,0 mm hingegen ist die Kapillarwirkung der Seele häufig nur gering ausgeprägt.

Diffusionsstäbchen mit einem Durchmesser der Seele von kleiner gleich 0,50 mm werden im Rahmen der vorliegenden Erfindung als Diffusionsstäbchen ohne Seele angesehen.

Es ist erfindungsgemäß bevorzugt, wenn die Seele an den Enden der Diffusionsstäbchen offen ist und der Durchmesser an den Enden maximal 50 % geringer ist als der durchschnittliche Durchmesser der Seele entlang des Diffusionsstäbchens, vorzugsweise maximal 25 % geringer, weiter bevorzugt maximal 10 % geringer. Nach dem Zusammenrollen des Papiers zu einem Diffusionsstäbchen müssen die Diffusionsstäbchen üblicherweise auf Länge geschnitten werden und durch den Schnitt kann es vorkommen, dass der Durchmesser der Seele an den Enden geringer ist als entlang des Diffusionsstäbchens. Es ist erfindungsgemäß nicht bevorzugt und zu vermeiden, dass die Enden des Diffusionsstäbchen verschlossen sind, obwohl eine Seele innerhalb des Diffusionsstäbchens ausgebildet ist.

Eine Verwendung ist erfindungsgemäß bevorzugt, wobei die Seele einen Durchmesser von mindestens 0,50 mm aufweist, vorzugsweise mindestens 0,60 mm aufweist, weiter bevorzugt mindestens 0,70 mm aufweist.

Eine Verwendung ist erfindungsgemäß bevorzugt, wobei die Seele einen Durchmesser von maximal 3,5 mm aufweist, vorzugsweise maximal 2,5 mm, weiter bevorzugt maximal 2,0mm.

Eine Verwendung ist erfindungsgemäß bevorzugt, wobei die Seele einen Durchmesser im Bereich von 0,50 mm bis 3,5 mm aufweist, vorzugsweise im Bereich von 0,60 mm bis 3,0 mm aufweist, weiter bevorzugt im Bereich von 0,70 mm bis 2,0 mm aufweist.

Eigene Untersuchungen haben gezeigt, dass bei einem Durchmesser der Diffusionsstäbchen von 4 mm 50 mL der Zusammensetzung die besten Verdampfungseigenschaften erhalten werden, wenn vier Diffusionsstäbchen mit 170 mm Länge eingesetzt werden. Bei einem Durchmesser der Diffusionsstäbchen von 4 mm 100 mL der Zusammensetzung werden die besten Verdampfungseigenschaften erhalten, wenn vier Diffusionsstäbchen mit 200 mm Länge eingesetzt werden. Bei einem Durchmesser der Diffusionsstäbchen von 4 mm 120 mL der Zusammensetzung werden die besten Verdampfungseigenschaften erhalten, wenn vier Diffusionsstäbchen mit 240 mm Länge eingesetzt werden. Bei einem Durchmesser der Diffusionsstäbchen von 4 mm 200 mL der Zusammensetzung werden die besten Verdampfungseigenschaften erhalten, wenn acht Diffusionsstäbchen mit 240 mm Länge eingesetzt werden. Es besteht allerdings die Möglichkeit kürzere Diffusionsstäbchen einzusetzen und gleichzeitig die Anzahl und/oder den Durchmesser der Diffusionsstächen zu erhöhen.

Es ist erfindungsgemäß bevorzugt, wenn sämtliche Diffusionsstäbchen identisch ausgestaltet sind und keine weiteren Diffusionsstäbchen enthalten sind. Alternativ können auch verschiedene Diffusionsstächen miteinander gemischt werden, so können beispielsweise Diffusionsstächen, die Fasern pflanzlicher Herkunft umfassen oder daraus bestehen, mit Diffusionsstäbchen gemischt werden, die Fasern tierischer Herkunft umfassen oder daraus bestehen.

Eine erfindungsgemäße Vorrichtung ist bevorzugt, wobei der die flüssige Zusammensetzung
5 bis 90 Gew.-%, vorzugsweise 6 bis 70 Gew.-%, besonders bevorzugt 8 bis 65 Gew.-% des mit Wasser mischbaren Lösungsmittels
1 bis 25 Gew.-% vorzugsweise 2 bis 20 Gew.-%, besonders bevorzugt 4 bis 10 Gew.-% ein oder mehrere Wirkstoffe
10 bis 90 Gew.-%, vorzugsweise 20 bis 85 Gew.-%, besonders bevorzugt 25 bis 85 Gew.-% Wasser
   und
0 bis 20 Gew.-% vorzugsweise 0,5 bis 15 Gew.-%, besonders bevorzugt 1,0 bis 10 Gew.-% Tensid
umfasst oder daraus besteht, jeweils bezogen auf das Gesamtgewicht der Zusammensetzung.

Eigene Untersuchungen haben gezeigt, dass es bevorzugt ist, wenn das Verhältnis zwischen sämtlichen Wirkstoffen und dem Tensid im Bereich zwischen 1 : 2 und 2 : 1 ist.

Eine Vorrichtung (1) zum Freisetzen eines Wirkstoffes in die Umgebung ist besonders bevorzugt bestehend aus oder umfassend
a) eine flüssige Zusammensetzung (5) bestehend aus oder umfassend
   a1) 10 bis 90 Gew.-% Ethanol und/oder Dipropylenglycolmethylether
   a2) 1 bis 25 Gew.-% eines oder mehrerer Wirkstoffe,
   a3) 10 bis 90 Gew.-% Wasser und
      und
   a4) 0 bis 10 Gew.-% Tensid, das einen nach Griffin berechneten HLB-Wert im Bereich von 3 bis 20 aufweist,
   bezogen auf das Gesamtgewicht der flüssigen Zusammensetzung
b) mindestens ein Diffusionsstäbchen (3, 3', 3") bestehend aus oder umfassend Zellstofffasern, natürlicher Stärke und ein oder mehrere Carbonate
   und
c) einen Behälter (2), der zumindest eine Behälteröffnung (4) aufweist und die flüssige Zusammensetzung (5) umfasst.

Ein weiter Aspekt der vorliegenden Erfindung betrifft ein Verfahren zur Herstellung einer Vorrichtung (1) zum Freisetzen eines Wirkstoffes umfassend die folgenden Schritte:
i) Herstellen oder Bereitstellen eines erfindungsgemäßen Kits,
ii) Überführen sämtlicher Komponenten der Zusammensetzung in einen Behälter, der zumindest eine Behälteröffnung (4) aufweist, und optionales Vermischen sämtlicher Komponenten,
   und
iii) Hinzufügen der Diffusionsstäbchen (3, 3', 3") durch die Behälteröffnung, sodass ein Teil der jeweiligen Diffusionsstäbchen (3, 3', 3") in Kontakt mit der Zusammensetzung befindet und ein Teil der jeweiligen Diffusionsstäbchen (3, 3', 3") sich durch die Behälteröffnung (4) aus dem Behälter erstreckt.

Ein weiterer Aspekt der vorliegenden Erfindung betrifft eine Verwendung von Diffusionsstäbchen in Vorrichtungen (1) zum Freisetzen eines Wirkstoffes, bei denen die den Wirkstoff umfassende Zusammensetzung
a1) ein mit Wasser mischbares Lösungsmittel,
a2) ein oder mehrere Wirkstoffe,
a3) Wasser
   und
optional a4) ein Tensid

umfasst oder daraus besteht
und wobei die Diffusionsstäbchen aus
   i) Fasern pflanzlicher Herkunft
      und/oder
   ii) Fasern tierischer Herkunft
bestehen oder diese umfassen.

Die Ausgestaltung des erfindungsgemäßen Kits bzw. der erfindungsgemäßen Vorrichtung, insbesondere im Hinblick auf die Diffusionsstäbchen und die Zusammensetzung, gelten analog für die erfindungsgemäße Verwendung. Dies gilt insbesondere für die als bevorzugt und besonders bevorzugt gekennzeichneten Ausgestaltungsformen.

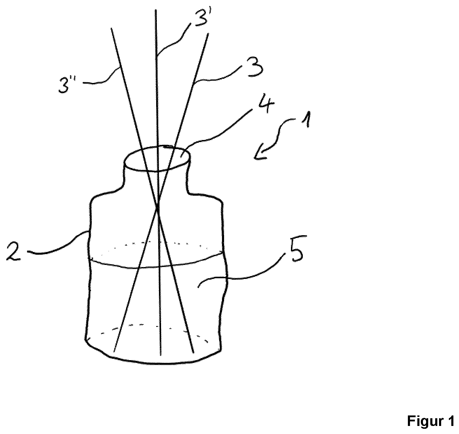

Die Erfindung ist nachstehend anhand einer Zeichnung beispielhaft erläutert. Diese zeigt in Fig.1 eine erfindungsgemäße Vorrichtung (1) zum Freisetzen eines Wirkstoffes in die Umgebung in einer schematischen Darstellung. In einem Behälter (2), der eine Behälteröffnung (4) aufweist, befindet sich eine flüssige Zusammensetzung (5). Die flüssige Zusammensetzung (5) umfasst Wasser, ein mit Wasser mischbares Lösungsmittel (Ethanol oder Dipropylenglycolmethylether) und ein oder mehrere Wirkstoffe (hier Duftstoffe). In dem Behälter sind drei Diffusionsstäbchen (3, 3', 3") angeordnet, die in Kontakt mit der in dem Behälter befindlichen flüssigen Zusammensetzung (5) stehen und sich durch die Behälteröffnung (4) aus dem Behälter (2) erstrecken. Bei den Diffusionsstäbchen (3, 3', 3") handelt es sich um Diffusionsstäbchen, die hautsächlich Zellstoffasern und zusätzlich natürliche Stärke und Carbonate als Füllstoffe umfassen.

### Beispiel 1:

Es wird eine Zusammensetzung erstellt, die 60 Gew.-% Dipropylenglycolmethylether, 30 Gew.-% Wasser und 10 Gew.-% Duftstoff umfasst. Diese Zusammensetzung wird in eine zylindrische Glasflasche überführt. Durch die Öffnung der Glasflasche werden vier Diffusionsstäbchen aus Zellstofffasern eingeführt, sodass die flüssige Zusammensetzung über die Diffusionsstäbchen aus der Glasflasche transportiert und an der Oberfläche der Diffusionsstäbchen verdampfen kann.

Die Duftintensität ist über die gesamte Laufzeit sehr gut.

### Beispiel 2:

Es wird eine Zusammensetzung erstellt, die 10 Gew.-% Ethanol, 80 Gew.-% Wasser, 5 Gew.-% Tensid und 5 Gew.-% Duftstoff umfasst. Diese Zusammensetzung wird in eine zylindrische Glasflasche überführt. Durch die Öffnung der Glasflasche werden vier Diffusionsstäbchen aus Zellstofffasern eingeführt, sodass die flüssige Zusammensetzung über die Diffusionsstäbchen aus der Glasflasche transportiert und an der Oberfläche der Diffusionsstäbchen verdampfen kann.

Die Duftintensität ist über die gesamte Laufzeit sehr gut.

## Patentansprüche

1. Kit zur Herstellung einer Vorrichtung (1) zum Freisetzen eines Wirkstoffes in die Umgebung, umfassend oder bestehend aus
a) eine Zusammensetzung bestehend aus oder umfassend
a1) ein mit Wasser mischbares Lösungsmittel
a2) ein oder mehrere Wirkstoffe
b) mindestens ein Diffusionsstäbchen (3, 3', 3") bestehend aus oder Fasern pflanzlicher Herkunft und wobei das oder die Diffusionsstäbchen durch Aufrollen von Papier hergestellt wurde(n).

2. Kit nach Anspruch 1, wobei das mit Wasser mischbare Lösungsmittel ein Alkohol oder ein Ether ist, vorzugsweise Ethanol oder Dipropylenglycolmethylether ist.

3. Kit nach einem der vorherigen Ansprüche, wobei die Zusammensetzung flüssig ist und alle Komponenten in dieser gelöst sind.

4. Kit nach einem der vorherigen Ansprüche, wobei die Fasern pflanzlicher Herkunft ausgewählt sind aus der Gruppe bestehend aus Zellstofffasern, Samenfasern, Bastfasern und Hartfasern.

5. Kit nach Anspruch 4, wobei der Massenanteil an Zellstofffasern im Bereich von 60,0 % bis 90,0 % liegt, vorzugsweise im Bereich von 70,0 % bis 85,0 % liegt, besonders bevorzugt im Bereich von 75,0 % bis 80,0 % liegt, bezogen auf die Gesamtmasse des Diffusionsstäbchen und bestimmt als lufttrockener Massenanteil (Lutro).

6. Kit nach einem der vorherigen Ansprüche, wobei das mindestens eine Diffusionsstäbchen (3, 3', 3") oder sämtliche Diffusionsstäbchen ein Bindemittel umfasst, wobei es sich bei dem Bindemittel vorzugsweise um Stärke handelt.

7. Kit nach einem der Ansprüche 4 bis 6, wobei es sich bei den Zellstofffasern um Fasern aus Kiefern-, Birken, Pappeln, Fichten-, Buchen- oder Eukalyptusholz handelt.

8. Kit nach einem der vorherigen Ansprüche, wobei die Diffusionsstäbchen einen Durchmesser im Bereich von 2 bis 10 mm aufweisen, vorzugsweise im Bereich von 3 bis 7 mm aufweisen, besonders bevorzugt im Bereich von 3 bis 5 mm aufweisen.

9. Kit nach einem der vorherigen Ansprüche, wobei das Diffusionsstäbchen (1) eine hohle Seele (5) entlang seiner Achse aufweist es sich bei dem Diffusionsstäbchen (1) um einen Hohlzylinder mit einem (Außen)Durchmesser, einem Durchmesser der Seele (Innendurchmesser) und einer Wanddicke handelt.

10. Kit nach einem der vorherigen Ansprüche, wobei das Kit zusätzlich
c) einen Behälter (2) umfasst, der zumindest eine Behälteröffnung (4) aufweist.

11. Kit nach einem der vorherigen Ansprüche, wobei die Zusammensetzung zusätzlich
a4) ein Tensid enthält.

12. Kit nach einem der vorherigen Ansprüche, wobei es sich bei dem Wirkstoff um einen Duftstoff handelt.

13. Vorrichtung (1) zum Freisetzen eines Wirkstoffes in die Umgebung hergestellt aus einem Kit nach einem der vorherigen Ansprüche.

14. Verfahren zur Herstellung einer Vorrichtung (1) zum Freisetzen eines Wirkstoffes umfassend die folgenden Schritte:
i) Herstellen oder Bereitstellen eines Kits nach einem der vorherigen Ansprüche,
ii) Überführen sämtlicher Komponenten der Zusammensetzung in einen Behälter, der zumindest eine Behälteröffnung (4) aufweist, und optionales Vermischen sämtlicher Komponenten,
und
iii) Hinzufügen der Diffusionsstäbchen (3, 3', 3") durch die Behälteröffnung, sodass ein Teil der jeweiligen Diffusionsstäbchen (3, 3', 3") in Kontakt mit der Zusammensetzung befindet und ein Teil der jeweiligen Diffusionsstäbchen (3, 3', 3") sich durch die Behälteröffnung (4) aus dem Behälter erstreckt.

15. Verwendung von Diffusionsstäbchen in Vorrichtungen (1) nach Anspruch 13 zum Freisetzen eines Wirkstoffes.

## Claims

1. Kit for producing a device (1) for releasing an active substance into the surroundings, comprising or consisting of
a) a composition consisting of or comprising
a1) a water-miscible solvent
a2) one or more active substances
b) at least one diffusion stick (3, 3', 3") consisting of or comprising fibres of plant origin, wherein the diffusion stick or sticks are produced by rolling paper.

2. Kit according to claim 1, wherein the water-miscible solvent is an alcohol or an ether, preferably ethanol or dipropylene glycol methyl ether.

3. Kit according to any one of the preceding claims, wherein the composition is liquid and all components are dissolved therein.

4. Kit according to any one of the preceding claims, wherein the fibres of plant origin are selected from the group consisting of cellulose fibres, seed fibres, bast fibres and hard fibres.

5. Kit according to claim 4, wherein the mass fraction of cellulose fibres is in the range from 60.0% to 90.0%, preferably in the range from 70.0% to 85.0%, particularly preferably in the range from 75.0% to 80.0%, based on the total mass of the diffusion stick and determined as the air-dry mass fraction (lutro).

6. Kit according to any one of the preceding claims, wherein the at least one diffusion stick (3, 3', 3"), or all diffusion sticks, comprise a binder, wherein the binder is preferably starch.

7. Kit according to any one of claims 4 to 6, wherein the cellulose fibres are fibres from pine, birch, poplar, spruce, beech or eucalyptus wood.

8. Kit according to any one of the preceding claims, wherein the diffusion sticks have a diameter in the range from 2 to 10 mm, preferably in the range from 3 to 7 mm, particularly preferably in the range from 3 to 5 mm.

9. Kit according to any one of the preceding claims, wherein the diffusion stick (1) has a hollow core (5) along its axis and the diffusion stick (1) is a hollow cylinder having an (outer) diameter, a core diameter (inner diameter) and a wall thickness.

10. Kit according to any one of the preceding claims, wherein the kit additionally c) comprises a container (2) having at least one container opening (4).

11. Kit according to any one of the preceding claims, wherein the composition additionally contains
a4) a surfactant.

12. Kit according to any one of the preceding claims, wherein the active substance is a fragrance.

13. Device (1) for releasing an active substance into the surroundings, produced from a kit according to any one of the preceding claims.

14. Method for producing a device (1) for releasing an active substance, comprising the following steps:
i) producing or providing a kit according to any one of the preceding claims,
ii) transferring all components of the composition into a container having at least one container opening (4), and optionally mixing all components,
and
iii) adding the diffusion sticks (3, 3', 3") through the container opening such that a portion of the respective diffusion sticks (3, 3', 3") is in contact with the composition and a portion of the respective diffusion sticks (3, 3', 3") extends out of the container through the container opening (4).

15. Use of diffusion sticks in devices (1) according to claim 13 for releasing an active substance.

## Revendications

1. Kit pour la fabrication d'un dispositif (1) destiné à libérer une substance active dans l'environnement, comprenant ou consistant en
a) une composition consistant en ou comprenant
a1) un solvant miscible avec l'eau
a2) une ou plusieurs substances actives
b) au moins un bâtonnet de diffusion (3, 3', 3") consistant en ou comprenant des fibres d'origine végétale, le ou les bâtonnets de diffusion étant obtenus par enroulement de papier.

2. Kit selon la revendication 1, dans lequel le solvant miscible avec l'eau est un alcool ou un éther, de préférence l'éthanol ou l'éther méthylique du dipropylène glycol.

3. Kit selon l'une quelconque des revendications précédentes, dans lequel la composition est liquide et tous les composants y sont dissous.

4. Kit selon l'une quelconque des revendications précédentes, dans lequel les fibres d'origine végétale sont choisies dans le groupe constitué de fibres de cellulose, fibres de graines, fibres libériennes et fibres dures.

5. Kit selon la revendication 4, dans lequel la fraction massique de fibres de cellulose se situe dans une plage de 60,0 % à 90,0 %, de préférence dans une plage de 70,0 % à 85,0 %, particulièrement de préférence dans une plage de 75,0 % à 80,0 %, par rapport à la masse totale du bâtonnet de diffusion et déterminée comme fraction massique à l'état sec à l'air (lutro).

6. Kit selon l'une quelconque des revendications précédentes, dans lequel ledit au moins un bâtonnet de diffusion (3, 3', 3"), ou tous les bâtonnets de diffusion, comprennent un liant, le liant étant de préférence de l'amidon.

7. Kit selon l'une quelconque des revendications 4 à 6, dans lequel les fibres de cellulose sont des fibres provenant de bois de pin, de bouleau, de peuplier, d'épicéa, de hêtre ou d'eucalyptus.

8. Kit selon l'une quelconque des revendications précédentes, dans lequel les bâtonnets de diffusion présentent un diamètre dans une plage de 2 à 10 mm, de préférence dans une plage de 3 à 7 mm, particulièrement de préférence dans une plage de 3 à 5 mm.

9. Kit selon l'une quelconque des revendications précédentes, dans lequel le bâtonnet de diffusion (1) présente une âme creuse (5) le long de son axe et le bâtonnet de diffusion (1) est un cylindre creux ayant un diamètre (extérieur), un diamètre de l'âme (diamètre intérieur) et une épaisseur de paroi.

10. Kit selon l'une quelconque des revendications précédentes, dans lequel le kit comprend en outre
c) un récipient (2) présentant au moins une ouverture de récipient (4).

11. Kit selon l'une quelconque des revendications précédentes, dans lequel la composition contient en outre
a4) un tensioactif.

12. Kit selon l'une quelconque des revendications précédentes, dans lequel la substance active est un parfum.

13. Dispositif (1) destiné à libérer une substance active dans l'environnement, obtenu à partir d'un kit selon l'une quelconque des revendications précédentes.

14. Procédé de fabrication d'un dispositif (1) destiné à libérer une substance active, comprenant les étapes suivantes :
i) fabrication ou mise à disposition d'un kit selon l'une quelconque des revendications précédentes,
ii) transfert de tous les composants de la composition dans un récipient présentant au moins une ouverture de récipient (4), et mélange optionnel de tous les composants,
et
iii) ajout des bâtonnets de diffusion (3, 3', 3") à travers l'ouverture du récipient, de sorte qu'une partie des bâtonnets de diffusion respectifs (3, 3', 3") est en contact avec la composition et qu'une partie des bâtonnets de diffusion respectifs (3, 3', 3") s'étend hors du récipient à travers l'ouverture du récipient (4).

15. Utilisation de bâtonnets de diffusion dans des dispositifs (1) selon la revendication 13 pour libérer une substance active.
